(19) Europäisches Patentamt

European Patent Office

Office européen des brevets

(11) **EP 4 301 422 B1**

(12) **EUROPEAN PATENT SPECIFICATION**

(45) Date of publication and mention
of the grant of the patent:
**11.02.2026 Bulletin 2026/07**

(21) Application number: **21709670.0**

(22) Date of filing: **02.03.2021**

(51) International Patent Classification (IPC):
**A61L 9/03** *(2006.01)*

(52) Cooperative Patent Classification (CPC):
**A61L 9/037**

(86) International application number:
**PCT/EP2021/055201**

(87) International publication number:
**WO 2022/184241 (09.09.2022 Gazette 2022/36)**

(54) **DEVICE AND METHOD FOR DISPENSING VOLATILE SUBSTANCES**

**VORRICHTUNG UND VERFAHREN ZUR ABGABE VON FLÜCHTIGEN SUBSTANZEN**

**DISPOSITIF ET PROCÉDÉ DE DISTRIBUTION DE SUBSTANCES VOLATILES**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**10.01.2024 Bulletin 2024/02**

(73) Proprietor: **CTR, Lda
2135-301 Samora Correia (PT)**

(72) Inventors:
• **DYCHER, David
Isle of Man (GB)**
• **VIEIRA, Pedro Queiroz
2605-197 Belas (PT)**
• **HISHIKI, Teruo
Tokyo, 166--0012 (JP)**

(74) Representative: **Liebl, Thomas
Neubauer - Liebl - Bierschneider - Massinger
Münchener Straße 49
85051 Ingolstadt (DE)**

(56) References cited:
**WO-A1-2017/215726     WO-A1-98/58692
WO-A2-2007/138246**

**EP 4 301 422 B1**

**Description**

The invention relates to a device for dispensing, in particular for vaporising, volatile substances, in particular fragrances and/or active substances, according to the preamble of claim 1. Furthermore, the invention relates to a method for dispensing, in particular for vaporising, volatile substances, in particular fragrances and/or active substances, according to the preamble of claim 15.

Devices for dispensing, in particular for vaporising, volatile substances, in particular fragrances and/or active substances, are generally known and generally comprise a device housing and a container in which a substance to be dispensed is accommodated and which is at least partially insertable into the housing. A wick is arranged in the container as a capillary element, which projects beyond the container with a free wick end and is in contact with the substance to be dispensed in such a way that this is conveyed towards the free wick end by means of the capillary action of the wick. A heating element, in particular an electrical heating element, is regularly associated with the free wick end, by means of which heat can be applied to the free wick end in order to be able to quickly release the substance accumulating in the free wick end into the environment or to evaporate it. Such a structure is known, for example, from WO 2017/215726 A1. In addition, it is generally known to adjust the degree of evaporation and thus the evaporation power by changing the heat transfer to the free wick end. For this purpose, it is generally known to adjust the electrical power supply of the heating element, in particular in three heating stages, low, medium and high. In addition, it is known from WO 98/58692 A1 that, in order to adjust the degree of evaporation and the evaporation output, the container together with the wick can be mounted in the housing of the device in a height-adjustable manner in such a way that the relative position of the wick end to the heating device and thus the heat transfer can be changed.

The evaporation rate and thus the delivery rate of the substance to be delivered can be influenced with the known individual and manual settings described above, although the following problems are associated with this:

If the heating power is set too low, the evaporation rate and thus the evaporation performance are low, so that the desired effect is only insufficiently achieved. In order to avoid this, users of the device very often select and set a high, usually the highest possible heating power from the outset. This can have the disadvantage of drying out and dehumidifying the free wick end to an inadmissibly high degree, which can damage the capillary structure in the wick end by sticking and/or baking together. Contrary to the user's intention, the set high heating power thus considerably reduces the evaporation rate and thus the dispensing rate of the substance to be dispensed and, in extreme cases, no substance is evaporated any more and a partially still filled container has to be replaced. This negative effect can be intensified by further unfavourable boundary conditions, in particular by high ambient temperatures and low humidity.

WO 2007/138246 A2 discloses an emanation device that comprises an emanation material container, an emanation section and emanation material level indication means. The emanation material level indication means comprises control means and a temperature sensor, which is adapted to sense a temperature of a wick. The change in wick temperature between a wet and a dry state is used to determine the fill level of the fragrance container.

In contrast, it is the object of the present invention to provide a device and a method for dispensing, in particular for vaporising, volatile substances, in particular fragrances and/or active substances, by means of which the substance delivery is made effective and stable, or a high degree of efficiency of the substance delivery is achieved, in particular also under changing boundary conditions.

This object is solved with the features of the independent patent claims. Advantageous embodiments thereof are the subject of the related subclaims.

According to claim 1, a device for dispensing, in particular for vaporising, volatile substances, in particular fragrances and/or active substances, is claimed, having a housing and having a container for the substance to be dispensed, which is at least partially insertable into the housing. The device further comprises a wick as a capillary element which is in contact with the substance to be dispensed, which wick is arranged at least partially in the container and forms part of the container, and which furthermore comprises a wick-side substance dispensing region. Furthermore, the device has at least one electrical heating element in the region of the wick-side substance dispensing region, by means of which a substance-enriched substance air flow can be generated by heat transfer to the substance dispensing region and flows out of the device and out of the housing.

According to the invention, the device comprises a temperature measuring device for direct or indirect measurement of the wick temperature at the wick-side substance dispensing region. This temperature measuring device has at least one temperature sensor and a measuring value output, at which an electrical wick temperature measuring signal corresponding to the currently measured wick temperature is output during operation.

Furthermore, the device has an open-loop control device or a closed-loop control device for controlling the wick temperature at the wick-side substance dispensing region, which in turn has a control module at which a wick temperature setpoint value, preferably in a functionally permissible setpoint value range without damaging the capillary structure of the wick end, can be set, preferably be preset and - fixed or variable - adjustable, by means of a setpoint value actuator and at which the current wick temperature measurement signal is input as an actual wick temperature value. By means of a comparator unit and a stored control algorithm, the heating power of the electric heating element is varied and adjusted to achieve and maintain the wick temperature setpoint value.

The invention is thus based on the knowledge gained that the capillary action of the free wick end is irreversibly damaged or, in extreme cases, destroyed if the free wick end is subjected to an impermissibly high temperature. With a setpoint adjustment in the range of a permissible wick temperature at which no damage or destruction of the capillary action at the free wick end occurs, an effective, long-term stable and uniform evaporation rate is achieved.

Direct measurement of the wick temperature at the wick-side substance delivery area is possible in principle, but technically difficult and/or cost-intensive e.g. due to limited installation space. Therefore, in the following embodiment, a suitable, indirect, simulated and very accurate measurement of the wick temperature at the wick-side substance delivery area is proposed:

For a particularly accurate measurement, it is taken into account that heat transfer to the wick-side substance delivery area for achieving and maintaining a wick temperature setpoint occurs on the one hand by thermal radiation (if necessary also by thermal conduction) from the heating element to the substance delivery area, but on the other hand also by convection of ambient air currently contained in the housing, which flows past the substance delivery area and carries thermal energy with it.

A temperature measuring device for the simulation and indirect measurement of the exact wick temperature at the wick-side substance dispensing region has at least two temperature sensors, preferably formed by thermistors. At least one temperature sensor (preferably one or two NTC temperature sensors as heating element temperature sensors) for measuring the heating element temperature is arranged on or in the heating element. In addition, at least one temperature sensor, preferably a (pre-calibrated) semiconductor temperature sensor, is arranged in the housing at a distance from the heating element as an ambient temperature sensor for measuring the ambient air temperature.

The temperature measuring device also has a function module, preferably in the form of a microcomputer unit (MCU), with a computing function and/or memory function, to which the at least two temperature sensors are connected for inputting the measured heating element temperature and the measured ambient air temperature. The high-precision wick temperature (TD) at the wick-side substance dispensing region is determined indirectly with the function module from the measured heating element temperature (TH) and the measured ambient air temperature (TU).

There is a functional relationship between the measured heating element temperature (TH) and the measured ambient air temperature (TU) to determine the wick temperature (TD):

$$TD = f(TH, TU)$$

This was determined in advance experimentally using a sample device of a device series, whereby a corresponding function is stored in each function module of the device series, in particular as an algorithm and/or as a characteristic diagram and/or as a characteristic curve. During operation of the device, a current wick temperature measurement signal is then determined and output at the function module (working as a wick temperature simulator) by evaluating the stored function, which signal can be further used as a wick temperature actual value signal.

For an often sufficiently accurate wick temperature determination, a simple linear equation can be used:

$$TD = f(TH, TU) = A \times TH + B \times TU + C,$$

where the quantities A, B, C can be determined experimentally in advance on at least one sample device, for example with a characteristic order of magnitude A = 0.9; B = 0.1; C = -10. To increase the accuracy of the wick temperature measurement, non-linear functions can also be stored in the function module.

To measure the ambient temperature in the housing, which is up to approx. 85°C as an example, a pre-calibrated semiconductor temperature sensor available on the market with a measuring range of approx. -10 to 85°C can be used. The operating temperature at the heating element, however, is considerably higher. For temperature measurement at the heating element, NTC temperature sensors are preferably used, which can withstand these higher temperatures, here for example with a measuring range of 0 to 160°C, without damage. However, temperature sensors, especially NTC temperature sensors, are usually individually different due to the manufacturing process and have, for example, strongly non-linear characteristic curves. For an accurate temperature measurement, the temperature sensors must therefore be calibrated. In the following, it is now indicated in general how such a calibration can be carried out relatively easily and with little effort in connection with the manufacture of the device:

First, the at least one heating element temperature sensor to be calibrated is combined with the pre-calibrated ambient temperature sensor in a calibration mode. For this purpose, the heating element is heated to several calibration temperature values which are measured with the pre-calibrated ambient temperature sensor. The corresponding electrical sensor values determined in combination are assigned to the calibration temperature values and stored in a memory of the function module, preferably with an interpolated characteristic curve.

In connection with the preferably used NTC temperature sensors, the following specific procedure results:

First, in a calibration mode, the NTC temperature sensor or sensors to be calibrated are measurably combined with the pre-calibrated semiconductor ambient temperature sensor. For this purpose, the heating element is heated to several calibration temperature values, preferably to three calibration temperature values of 30°C, 55°C and 80°C, these temperature values being measured with the pre-calibrated semiconductor temperature sensor. The combined determined corresponding electrical NTC sensor values are assigned to the calibration temperature values and stored in a memory of the function module, preferably in an interpolated NTC characteristic curve for the entire measuring range, which is used in the heating element temperature measurement.

In principle, to achieve and maintain a wick temperature setpoint value, control without feedback is possible. For an exact keeping of the wick temperature at the substance dispensing region, a control with feedback is preferably used, which particularly preferably has a control algorithm as PID controller with a combination of a proportional behaviour, an integral behaviour and a differential behaviour. In this case, the specified wick temperature setpoint value is compared with the measured actual wick temperature value and a manipulated/actuating variable is formed on the output side according to the control algorithm and previously experimentally determined control parameters, which is fed to a controllable actuator for the heating element. In a particularly preferred embodiment, at least one thyristor in the form of a triac with phase angle control is used as the actuator in the AC supply of the electrical heating element. The actuating variable is used to vary the phase angle control of the triac and thus the heating power of the heating element in a controlled manner to achieve and maintain the wick temperature setpoint value. A typical heating power of the heating element is between 3 to 5 Watt.

The control can be operated continuously with continuous adjustment of the heating power over time. To reduce the required amount of data and computing power, the control is preferably operated discontinuously, whereby the control algorithm can be activated discontinuously at relatively short time intervals, preferably in the range of seconds, for the evaluation of determined increments of the control feedback. Such a discontinuous control leads here only to small and thus tolerable deviations, since the control action only has a very slow effect on the wick temperature.

For switching the device on and off, a usual switch or push-button can be provided on the housing. Alternatively, connection to a power source, for example plugging in and out of a socket, can be used for this purpose.

In a simple embodiment, for example, a wick temperature setpoint value can be fixed by the manufacturer. In order to increase efficient operation and for individual adaptation of the evaporation rate and/or for adaptation to differently used substances, it is proposed to make the wick temperature setpoint value variable and adjustable within a permissible range. In a simpler embodiment, this can be carried out by means of a manually operable setting device attached to the housing, preferably as a switch and/or push-button.

In a particularly advantageous embodiment, the device has a radio module, preferably as a WiFi module, for a bidirectional, wireless data connection between the device and an external display and control device, preferably a smartphone with an associated app.

This allows a currently set wick temperature setpoint value and/or a current wick temperature actual value and/or a possibly currently set switch-on period of the device as well as possibly further information to be displayed on a screen on the display and control device.

On the other hand, the display and control unit can be used to transmit commands to the device, such as a wick temperature setpoint value adapted to a substance currently to be evaporated. This can be done, for example, by manually entering a code indicated on a substance package or a substance container, in particular a QR code, via a keyboard or by reading it by means of a scanner, i.e. transmitting it to the device.

Alternatively or additionally, switch-on times and/or further setting commands can be given to the device with a timer function. This enables convenient handling of the device with variable settings.

The device can have a mechanical structure known in principle, wherein the heating element is annular with a central wick-receiving opening for the wick, in which the wick projects at least partially with its substance-dispensing region, preferably without contact with a gap distance from the receiving opening, and wherein the substance-dispensing region of the wick lies in the housing in the vicinity of a substance-airflow outlet opening, from which the substance-airflow flows to the outside of the housing.

The wick-side substance dispensing region is formed by a free wick end projecting beyond the container, in particular a container opening, in such a way that the heating element is associated with the wick in the region of the free wick end.

The device is preferably designed as a plug component, in particular for small component dimensions and for ease of handling, and has plug contacts projecting from the housing, which can be plugged into an electrical socket for holding and for electrical power supply, preferably with alternating voltage. An adaptation to the AC voltage and frequency available in a region of use can be set in a fixed manner, for example for 230V AC voltage and 50Hz, or can be designed to be preselectable in a manner known per se.

Heating elements are generally known in different designs. Preferably, the heating element is intended here to be an electrical heating element which has a heating body made of a thermally conductive material and at least one electrical resistance element (for example in the form of a heating coil) which is thermally coupled to the heating body, preferably at least one electrical resistance element which is partially integrated and/or embedded in the heating body, and which can be connected to an energy source for the purpose of energy supply and/or can be supplied with electrical energy by means of an energy source.

The advantages resulting from the claimed method according to the invention correspond analogously to those of the device according to the invention, so that reference is made to the previously made explanations in order to avoid repetition.

The advantageous embodiments and further embodiments of the invention explained above and/or reproduced in the subclaims can be used individually or in any combination with one another - except, for example, in cases of clear dependencies or incompatible alternatives.

The invention and its advantageous embodiments and further embodiments are explained in more detail below with reference only to exemplary and schematic drawings.

They show:

Figure 1      shows a fully assembled evaporation device,

Figure 2      shows the evaporation device according to Figure 1 with the upper part of the housing removed,

Figure 3      the evaporation device according to Figure 2 with the lower part of the housing removed,

Figure 4      a longitudinal section along line A-A of Figure 2, and

Figure 5      a block diagram.

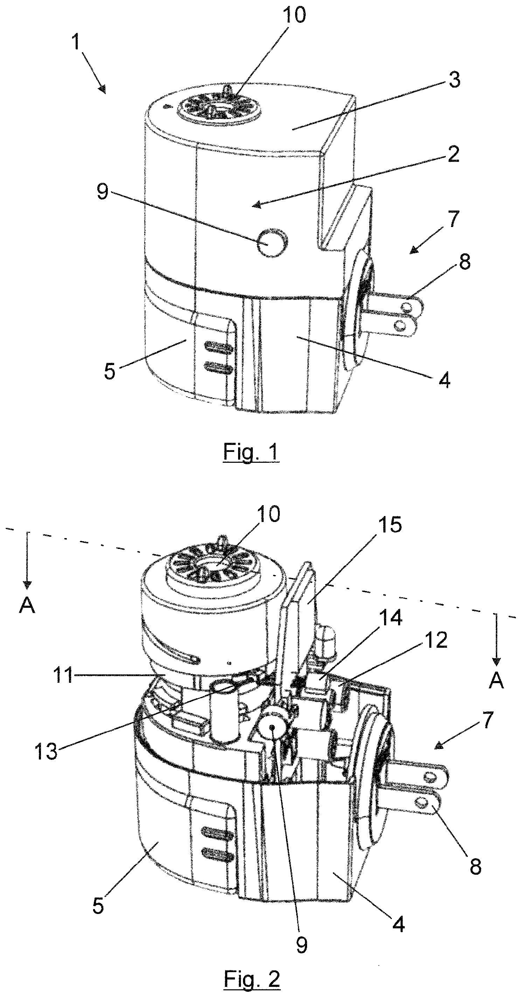

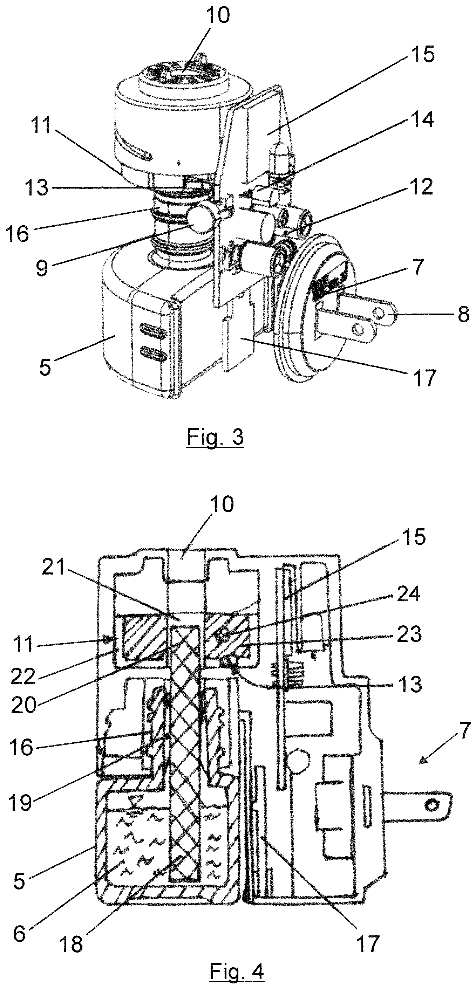

Figure 1 shows an evaporation device 1 as a plug-in component which has a housing 2 formed from a housing upper part 3 and a housing lower part 4. As can be seen from Figure 4, a container 5 is inserted into the housing 2 from below, in which a substance 6 to be vaporised is contained. Opposite the container 5 is a plug 7 with plug contacts 8 for insertion into an electrical socket, which serves here as an example as a holder for the vaporisation device 1 and for a power supply with alternating current.

In addition, an operating button 9, which can be operated from the outside, is arranged on the housing 2 for switching the vaporisation device 1 on and off and, if necessary, for selecting further functions. An exemplary outlet opening 10 is provided on the upper side of the evaporation device 1, from which a substance air flow enriched with the evaporated substance can escape.

In Figure 2, the upper part of the housing 3 has been removed, revealing part of the internal structure of the evaporation device 1. In the upper area, the outlet opening 10 is again visible, with an only partially visible annular heating element 11, the structure of which can be seen in Figure 4, and the temperature TH of which is controlled by means of a controller module 12.

To determine a wick temperature TD as the actual wick temperature value, which is fed to the controller module 12, a heating element temperature sensor 13 is arranged on the heating element 11 and, at a distance from it, an ambient temperature sensor 14 is arranged in the housing 2. Also shown in Figure 2 is the plug 7, which is electrically connected to downstream electrical components of the device 1. In addition, a radio module 15, for example in the form of a WiFi module, is mounted in the housing 2, for example for a bidirectional radio connection with a smartphone (not shown).

In Figure 3, both the upper part of the housing 3 and the lower part of the housing 4 have been removed, showing the container 5 with its semi-cylindrical shape and its container neck 16, to which a (unspecified) level sensor 17 is assigned. Figure 4 shows a partially schematised cross-sectional view as a central longitudinal section along the line A-A of Figure 2, showing that the container 5 holds both the substance to be vaporised and a wick 18 which extends upwards through the container neck 16 and a wick holder 19 and projects with a free wick end as a substance dispensing region 20 into a central wick receiving opening 21 of the ring-shaped heating element 11.

The heating element 11 has a heating element housing 22 in which an electrical resistance element 24 (shown schematically), for example a heating coil, is embedded in a thermally conductive potting material 23. The heating element temperature sensor 13 is also arranged on the heating element 11.

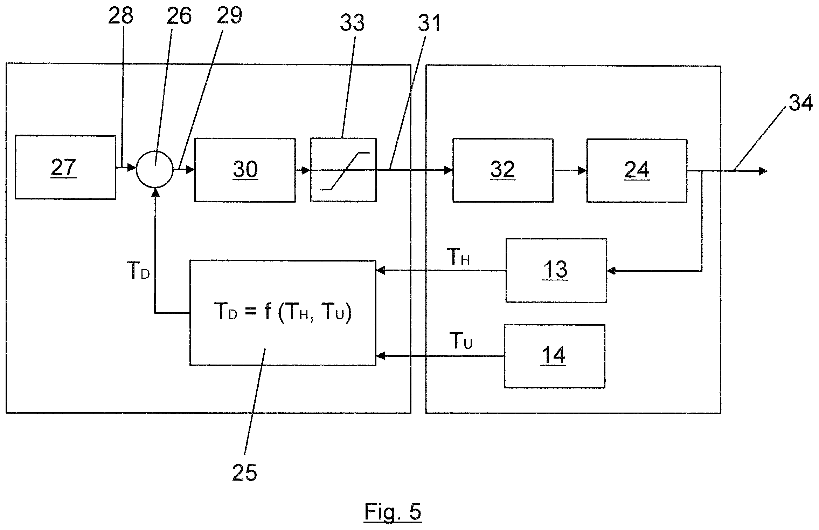

A presettable wick temperature as wick temperature setpoint value 38 at the free wick end 20 is controlled according to the block diagram in Figure 5:
The heating element temperature sensor 13 detects the heating element temperature TH and the ambient air temperature TU in the housing 2 is detected by the ambient temperature sensor 14. Both temperature measurement values are supplied to a function module 25, which evaluates the two supplied temperature measurement values using a stored function to simulate and indirectly determine the wick temperature TD at the end of the wick. The wick temperature TD calculated in this way is fed to a comparator unit 26 of the control device as the current actual wick temperature value. A wick temperature setpoint value 28 set there is also fed to the comparator unit 26 as a signal from a setpoint actuator 27.

A control deviation signal 29 is then formed in the comparator unit 26, which is fed to a PID controller 30, which, in accordance with its control algorithm and stored control parameters, outputs an actuating variable 31 as an actuating signal to a heating actuator 32, which is designed here as an example as a triac with phase angle control. The actuating variable 31 is also limited here to permissible values by a signal limiter 33 to prevent inadmissible overshooting of the control. The electrical resistance element 24 of the heating element 11 is connected downstream of the actuator 32, whereby the heat output (shown schematically by arrow 34) of the heating element 11 is controlled in such a way that the wick temperature setpoint value 28 is set and maintained.

REFERENCE LIST

1    evaporation device
2    housing
3    upper part of housing
4    lower part of housing
5    container
6    substance
7    plug
8    plug contact
9    operating button
10    outlet opening
11    heating element
12    control module
13    heating element temperature sensor
14    ambient temperature sensor
15    radio module
16    container neck
17    level sensor
18    wick
19    wick holder
20    wick end
21    wick holder opening
22    heating element housing
23    potting material
24    electrical resistance element
25    function module
26    comparator unit
27    setpoint actuator
28    wick temperature setpoint value
29    control deviation
30    PID controller
31    actuating variable
32    heating actuator
33    signal limiter
34    arrow

**Claims**

1.    Device for dispensing volatile substances (6),

with a housing (2),
with a container (5) for the substance (6) to be dispensed, which is at least partially insertable into the housing (2),
with a wick (18) as capillary element which is in contact with the substance (6) to be dispensed, and which is arranged at least partially in the container (5) and forms part of the container (5), and which has a wick-side substance-dispensing region (20),
with at least one electrical heating element (11) in the region of the wick-side substance-dispensing region (20), by means of which a substance-air flow which is enriched with substance (6) and which flows away from the device (1) and out of the housing (2) is generatable by means of heat transfer to the substance-dispensing region (20), **characterized**

**in that** the device (1) has a temperature measuring device for direct or indirect measurement of the wick temperature (TD) at the wick-side substance-dispensing region (20), having at least one temperature sensor (13, 14) and having a measuring value output at which, during operation, an electrical wick temperature measuring signal corresponding to the currently measured wick temperature (TD) is supplied,

**in that** the device (1) has an open- loop control device or a closed-loop control device for controlling the wick temperature (TD) at the wick-side substance dispensing region (20), and

**in that** the control device comprises a control module (30) at which a wick temperature setpoint value (28) is set by means of a setpoint value actuator (27) and at which the actual wick temperature measurement signal is input as the actual wick temperature value (TD), and which varies and adapts the heating power of the electric heating element (11) algorithm by means of a comparator unit (26) and a stored control algorithm in order to achieve and maintain the wick temperature setpoint value (28).

2. Device according to claim 1, **characterized:**

**in that** the temperature measuring device for simulating and indirectly measuring the wick temperature (TD) at the wick-side substance-dispensing region (20) has at least two temperature sensors (13, 14), preferably formed by thermistors,

**in that** at least one temperature sensor, preferably one or two NTC temperature sensors, is arranged as a heating element temperature sensor (13) for measuring the heating element temperature (TH) on or in the heating element (11),

**in that** at least one temperature sensor, preferably at least one semiconductor temperature sensor, is arranged as an ambient temperature sensor (14) for measuring the ambient air in the housing (2) spaced apart from the heating element (11) in the housing (2),

**in that** the temperature measuring device has at least one function module (25) as a simulation module, preferably as a microcomputer unit, with a computing function and/or memory function, to which the at least two temperature sensors (13, 4) for inputting the measured heating element temperature (TH) and the measured ambient air temperature (TU) are connected,

**in that** the wick temperature (TD) at the wick-side substance dispensing region (20) is indirectly determinable from the measured heating element temperature (TH) and the measured ambient air temperature (TU) by means of the function module, preferably in such a manner that the functional relationship between the heating element temperature (TH) and the ambient air temperature (TU) for the determination of the wick temperature (TD)

$$TD = f(TH, TU)$$

has been determined experimentally in advance on the basis of at least one sample device (1) of a device series and a corresponding function, in particular as an algorithm and/or as a characteristic diagram and/or as a characteristic curve, is stored in each function module (25) of the device series, and

**in that**, during operation of the device (1) and by evaluating the stored function, a current wick temperature measurement signal, which is available as a wick temperature actual value signal (TD), is determined and output at or with the function module (15) forming a wick temperature simulator.

3. Device according to claim 2, **characterized in that** the wick temperature (TD) is determined as a function of the heating element temperature (TH) and the ambient air temperature (TU) in the function module (25) according to the equation

$$TD = f(TH, TU) = A * TH + B * TU + C$$

with the variables A, B, C being determined experimentally in advance with the at least one sample device (1).

4. Device according to claim 2 or claim 3, **characterized**
**in that** the at least one heating element temperature sensor (13), preferably the heating element temperature sensor (13) formed by at least one NTC temperature sensor, is calibrateable, in particular during the manufacturing of the device (1), in such a way:

that the at least one heating element temperature sensor (13) to be calibrated is combined in a calibration mode with the pre-calibrated ambient temperature sensor (14), in particular with a semiconductor temperature sensor as pre-calibrated ambient temperature sensor (14),

that the heating element (11) is heated to a plurality of calibration temperature values which are measured with the pre-calibrated ambient temperature sensor (14), and

that the corresponding electrical sensor values which are determined in combination, preferably NTC sensor values, are assigned to the calibration temperature values and stored in a memory of the function module (25), preferably with an interpolated characteristic curve, most preferably with an interpolated NTC characteristic curve for the entire measuring range.

5. Device according to one of the preceding claims, **characterized**

**in that** the control module has a control algorithm, preferably in the form of a PID controller (30) with a combination of a proportional response and an integral response and a differential response, with the predetermined wick temperature setpoint value (28) being compared with the wick temperature actual value (TD) in a comparator unit (26) and an actuating variable (31) being formed on the output side in accordance with the control algorithm and regulating parameters determined experimentally in advance, and

**in that** the actuating variable (31) is fed to a controllable actuator (32) for the heating element (11), preferably in such way that the actuator is at least one thyristor in the form of a triac with phase-angle control in the AC supply of the electric heating element (11), so that the phase-angle control of the triac and thus the heating power of the heating element (11) is variable in a controlled manner with the actuating variable (31) in order to achieve and maintain the wick temperature setpoint (28).

6. Device according to claim 5, **characterized in that** the control algorithm is activatable discontinuously at predetermined time intervals, preferably in the range of seconds, for evaluating detected increments of the feedback of the closed-loop control.

7. Device according to one of the preceding claims, **characterized in that** a manually operable setting device, preferably in the form of a switch and/or push button (9), for switching on/off and/or for setting different wick temperature setpoints values (28) is arranged on the housing (2).

8. Device according to one of the preceding claims, **characterized in that** the device (1) has a radio module, preferably a WiFi module (15) as radio module, for a bidirectional and wireless data connection between the device (1) and an external display and control device, preferably a smartphone with associated app.

9. Device according to claim 8, **characterized in that** a currently set wick temperature setpoint value (28) and/or a current wick temperature actual value (TD) and/or a currently set switch-on period of the device and/or optionally further information can be displayed on a screen on the display and control device.

10. Device according to claim 8 or claim 9, **characterized in that** switch-on times are presettable with a timer function and/or **in that** a wick temperature setpoint value (28) adapted to a substance (6) to be currently evaporated is settable with the display and control unit of the device (1) via the wireless data connection, preferably in such way that a code indicated on a substance packaging or a substance container (5), in particular a QR code, is entered manually via a keyboard or is read in by means of a scanner, and/or **in that** switch-on times can be preset with a timer function,.

11. Device according to one of the preceding claims, **characterized In that** the heating element (11) is annular with a central wick-receiving opening (21) for the wick (18), in which the wick (18) projects at least partially with its substance-dispensing region (20), and

**in that** the substance dispensing region (20) of the wick (18) is located in the housing (2) in the region of a substance air flow outlet opening (10) from which the substance air flow flows to the outside of the housing (2).

12. Device according to claim 11, **characterized in that** the wick-side substance dispensing region (20) is formed by a free wick end projecting beyond the container (5), in particular a container opening (16), in such a way that the heating element (11) is assigned to the wick (18) in the region of the free wick end.

13. Device according to one of the preceding claims, **characterized in that** the device (1) is designed as a plug component with plug contacts (8) projecting from the housing (2), which plug contacts (8) are pluggable into an electrical socket for holding and for electrical power supply, preferably with alternating voltage.

**14.** Device according to one of the preceding claims, **characterized in that** the at least one heating element is an electrical heating element (11) which has a heating body comprising a heating element housing (22) and a thermally conductive material (23) and at least one electrically resistive element (24), in particular as a heating coil, which is thermally coupled to the heating body (11), preferably at least partially integrated and/or embedded in the heating body (11), and which is connectable to an energy source for the energy supply and/or is providable with electrical energy by means of an energy source.

**15.** Method for dispensing volatile substances (6),

with a device (1) with a housing (2),
with a container (5) for the substance (6) to be dispensed, which is at least partially insertable into the housing (2),
with a wick (18) as capillary element, which is in contact with the substance (6) to be dispensed and which is arranged at least partially in the container (5) and forms part of the container (5) and which has a wick-side substance-dispensing region (20),
with at least one electrical heating element (11) in the region of the wick-side substance-dispensing region (20), by means of which a substance-air flow which is enriched with substance (6) and which flows away from the device (1) and out of the housing (2) is generatable by means of heat transfer to the substance-dispensing region (20), **characterized**
**in that** the wick temperature (TD) at the wick-side substance dispensing region (20) is measured directly or indirectly with a temperature measuring device by means of at least one temperature sensor (13, 14), and an electrical wick temperature measuring signal corresponding to the current wick temperature (TD) is output at a measuring value output of the temperature measuring device during operation,
**in that** the wick temperature (TD) at the wick-side substance delivery region (20) is controlled with an open- loop control device or a closed-loop control device, and
**in that** the control device comprises a control module (30) at which a wick temperature setpoint value (28) is set with a setpoint value actuator (27) and at which the current wick temperature measuring signal is input as the actual wick temperature value (TD), and which varies and adapts the heating power of the electric heating element (11) by means of a comparator unit (26) and a stored control algorithm in order to achieve and maintain the wick temperature setpoint value (28).

## Patentansprüche

**1.** Vorrichtung zum Abgeben flüchtiger Substanzen (6),

mit einem Gehäuse (2),
mit einem Behälter (5) für die abzugebende Substanz (6), der zumindest teilweise in das Gehäuse (2) einsetzbar ist,
mit einem Docht (18) als Kapillarelement, der mit der abzugebenden Substanz (6) in Kontakt steht und der zumindest teilweise in dem Behälter (5) angeordnet ist und einen Teil des Behälters (5) bildet, und der einen dochtseitigen Substanzabgabebereich (20) aufweist,
mit zumindest einem elektrischen Heizelement (11) im Bereich des dochtseitigen Substanzabgabebereichs (20), mittels dem ein mit Substanz (6) angereicherter Substanz-Luftstrom, der von der Vorrichtung (1) weg und aus dem Gehäuse (2) herausströmt, mittels Wärmeübertragung auf den Substanzabgabebereich (20) erzeugbar ist, **dadurch gekennzeichnet,**
**dass** die Vorrichtung (1) eine Temperaturmesseinrichtung zur direkten oder indirekten Messung der Dochttemperatur (TD) am dochtseitigen Substanzabgabebereich (20) aufweist, mit zumindest einem Temperatursensor (13, 14) und mit einem Messwertausgang, an dem während des Betriebs ein elektrisches Dochttemperatur-Messsignal entsprechend der aktuell gemessenen Dochttemperatur (TD) bereitgestellt wird,
**dass** die Vorrichtung (1) eine Steuereinrichtung oder eine Regeleinrichtung zum Steuern oder Regeln der Dochttemperatur (TD) am dochtseitigen Substanzabgabebereich (20) aufweist, und
**dass** die Steuer- oder Regeleinrichtung ein Steuer- oder Regelmodul (30) umfasst, an dem ein Dochttemperatur-Sollwert (28) mittels eines Sollwertstellgliedes (27) eingestellt wird und an dem das tatsächliche Dochttemperatur-Messsignal als Ist-Dochttemperaturwert (TD) eingegeben wird, und welches die Heizleistung des elektrischen Heizelements (11) algorithmisch mittels einer Vergleichereinheit (26) und eines gespeicherten Steuer- oder Regelalgorithmus variiert und anpasst, um den Dochttemperatur-Sollwert (28) zu erreichen und aufrechtzuerhalten.

2. Vorrichtung nach Anspruch 1, **dadurch gekennzeichnet:**

**dass** die Temperaturmesseinrichtung zum Simulieren und indirekten Messen der Dochttemperatur (TD) am dochtseitigen Substanzabgabebereich (20) zumindest zwei Temperatursensoren (13, 14) aufweist, vorzugsweise gebildet durch Thermistoren,

**dass** zumindest ein Temperatursensor, vorzugsweise ein oder zwei NTC-Temperatursensoren, als Heizelement-Temperatursensor (13) zum Messen der Heizelementtemperatur (TH) an oder in dem Heizelement (11) angeordnet ist,

**dass** zumindest ein Temperatursensor, vorzugsweise zumindest ein Halbleiter-Temperatursensor, als Umgebungstemperatursensor (14) zum Messen der Umgebungsluft im Gehäuse (2), beabstandet von dem Heizelement (11) im Gehäuse (2), angeordnet ist,

**dass** die Temperaturmesseinrichtung zumindest ein Funktionsmodul (25) als Simulationsmodul, vorzugsweise als Mikrocomputereinheit, mit einer Rechenfunktion und/oder Speicherfunktion aufweist, an das die zumindest zwei Temperatursensoren (13, 14) zum Eingeben der gemessenen Heizelementtemperatur (TH) und der gemessenen Umgebungslufttemperatur (TU) angeschlossen sind,

**dass** die Dochttemperatur (TD) am dochtseitigen Substanzabgabebereich (20) aus der gemessenen Heizelementtemperatur (TH) und der gemessenen Umgebungslufttemperatur (TU) mittels des Funktionsmoduls indirekt bestimmbar ist, vorzugsweise derart, dass der funktionale Zusammenhang zwischen der Heizelementtemperatur (TH) und der Umgebungslufttemperatur (TU) zur Bestimmung der Dochttemperatur (TD)

$$TD = f(TH, TU)$$

im Voraus experimentell auf Basis zumindest einer Mustervorrichtung (1) einer Vorrichtungsserie bestimmt worden ist und eine entsprechende Funktion, insbesondere als Algorithmus und/oder als Kennfeld und/oder als Kennlinie, in jedem Funktionsmodul (25) der Vorrichtungsserie gespeichert ist, und

**dass** während des Betriebs der Vorrichtung (1) und durch Auswerten der gespeicherten Funktion ein aktuelles Dochttemperatur-Messsignal, das als Dochttemperatur-Istwertsignal (TD) vorliegt, bestimmt und an oder mit dem Funktionsmodul (15), das einen Dochttemperatursimulator bildet, ausgegeben wird.

3. Vorrichtung nach Anspruch 2, **dadurch gekennzeichnet, dass** die Dochttemperatur (TD) als Funktion der Heizelementtemperatur (TH) und der Umgebungslufttemperatur (TU) in dem Funktionsmodul (25) gemäß der Gleichung

$$TD = f(TH, TU) = A * TH + B * TU + C$$

bestimmt wird, wobei die Variablen A, B, C im Voraus experimentell mit der zumindest einen Mustervorrichtung (1) bestimmt werden.

4. Vorrichtung nach Anspruch 2 oder Anspruch 3, **dadurch gekennzeichnet,**
**dass** der zumindest eine Heizelement-Temperatursensor (13), vorzugsweise der Heizelement-Temperatursensor (13), der durch zumindest einen NTC-Temperatursensor gebildet ist, kalibrierbar ist, insbesondere während der Herstellung der Vorrichtung (1), derart:

**dass** der zumindest eine zu kalibrierende Heizelement-Temperatursensor (13) in einem Kalibrierungsmodus mit dem vorkalibrierten Umgebungstemperatursensor (14), insbesondere mit einem Halbleiter-Temperatursensor als vorkalibriertem Umgebungstemperatursensor (14), kombiniert wird,

**dass** das Heizelement (11) auf eine Mehrzahl von Kalibrierungstemperaturwerten aufgeheizt wird, die mit dem vorkalibrierten Umgebungstemperatursensor (14) gemessen werden, und

**dass** die entsprechenden elektrischen Sensorwerte, die in Kombination bestimmt werden, vorzugsweise NTC-Sensorwerte, den Kalibrierungstemperaturwerten zugeordnet und in einem Speicher des Funktionsmoduls (25) gespeichert werden, vorzugsweise mit einer interpolierten Kennlinie, besonders bevorzugt mit einer interpolierten NTC-Kennlinie für den gesamten Messbereich.

5. Vorrichtung nach einem der vorhergehenden Ansprüche, **dadurch gekennzeichnet,**

**dass** das Steuer- oder Regelmodul einen Steuer- oder Regelalgorithmus aufweist, vorzugsweise in Form eines PID-Reglers (30) mit einer Kombination aus einem proportionalen Verhalten und einem integralen Verhalten und einem differentialen Verhalten, wobei der vorbestimmte Dochttemperatur-Sollwert (28) mit dem Dochttemperatur-Istwert (TD) in einer Vergleichereinheit (26) verglichen wird und ausgangsseitig eine Stellgröße (31) gemäß dem Steuer- oder Regelalgorithmus und im Voraus experimentell bestimmten Regelparametern gebildet wird, und

**dass** die Stellgröße (31) einem steuerbaren Stellglied (32) für das Heizelement (11) zugeführt wird, vorzugsweise derart, dass das Stellglied zumindest ein Thyristor in Form eines Triacs mit Phasenanschnittsteuerung in der Wechselstromversorgung des elektrischen Heizelements (11) ist, so dass die Phasenanschnittsteuerung des Triacs und damit die Heizleistung des Heizelements (11) mit der Stellgröße (31) in gesteuerter Weise veränderbar ist, um den Dochttemperatur-Sollwert (28) zu erreichen und aufrechtzuerhalten.

6. Vorrichtung nach Anspruch 5, **dadurch gekennzeichnet, dass** der Steuer- oder Regelalgorithmus diskontinuierlich zu vorbestimmten Zeitintervallen, vorzugsweise im Bereich von Sekunden, aktivierbar ist, zum Auswerten erfasster Inkremente der Rückführung der Regeleinrichtung.

7. Vorrichtung nach einem der vorhergehenden Ansprüche, **dadurch gekennzeichnet, dass** eine manuell betätigbare Einstelleinrichtung, vorzugsweise in Form eines Schalters und/oder Druckknopfes (9), zum Ein- und Ausschalten und/oder zum Einstellen verschiedener Dochttemperatur-Sollwerte (28) an dem Gehäuse (2) angeordnet ist.

8. Vorrichtung nach einem der vorhergehenden Ansprüche, **dadurch gekennzeichnet, dass** die Vorrichtung (1) ein Funkmodul, vorzugsweise ein WLAN-Modul (15) als Funkmodul, für eine bidirektionale und drahtlose Datenverbindung zwischen der Vorrichtung (1) und einer externen Anzeige- und Bedieneinrichtung, vorzugsweise einem Smartphone mit zugehöriger App, aufweist.

9. Vorrichtung nach Anspruch 8, **dadurch gekennzeichnet, dass** ein aktuell eingestellter Dochttemperatur-Sollwert (28) und/oder ein aktueller Dochttemperatur-Istwert (TD) und/oder eine aktuell eingestellte Einschaltzeitdauer der Vorrichtung und/oder optional weitere Informationen auf einem Bildschirm auf der Anzeige- und Bedieneinrichtung angezeigt werden können.

10. Vorrichtung nach Anspruch 8 oder Anspruch 9, **dadurch gekennzeichnet, dass** Einschaltzeiten mit einer Timerfunktion voreinstellbar sind und/oder dass ein an eine Substanz (6), die aktuell zu verdampfen ist, angepasster Dochttemperatur-Sollwert (28) mit der Anzeige- und Bedieneinheit der Vorrichtung (1) über die drahtlose Datenverbindung einstellbar ist, vorzugsweise derart, dass ein auf einer Substanzverpackung oder einem Substanzbehälter (5) angegebener Code, insbesondere ein QR-Code, manuell über eine Tastatur eingegeben oder mittels eines Scanners eingelesen wird, und/oder dass Einschaltzeiten mit einer Timerfunktion voreingestellt werden können.

11. Vorrichtung nach einem der vorhergehenden Ansprüche, **dadurch gekennzeichnet,**

**dass** das Heizelement (11) ringförmig ist, mit einer zentralen dochtaufnehmenden Öffnung (21) für den Docht (18), in welche der Docht (18) zumindest teilweise mit seinem Substanzabgabebereich (20) hineinragt, und
**dass** der Substanzabgabebereich (20) des Dochtes (18) im Gehäuse (2) im Bereich einer Substanz-Luftstrom-Austrittsöffnung (10) angeordnet ist, aus der der Substanz-Luftstrom nach außen aus dem Gehäuse (2) strömt.

12. Vorrichtung nach Anspruch 11, **dadurch gekennzeichnet, dass** der dochtseitige Substanzabgabebereich (20) durch ein freies Dochtende gebildet ist, das über den Behälter (5), insbesondere eine Behälteröffnung (16), hinausragt, derart, dass das Heizelement (11) dem Docht (18) im Bereich des freien Dochtendes zugeordnet ist.

13. Vorrichtung nach einem der vorhergehenden Ansprüche, **dadurch gekennzeichnet, dass** die Vorrichtung (1) als Steckbauteil mit aus dem Gehäuse (2) herausragenden Steckkontakten (8) ausgebildet ist, welche Steckkontakte (8) in eine elektrische Steckdose zum Halten und zur elektrischen Energieversorgung einsteckbar sind, vorzugsweise mit Wechselspannung.

14. Vorrichtung nach einem der vorhergehenden Ansprüche, **dadurch gekennzeichnet, dass** das zumindest eine Heizelement ein elektrisches Heizelement (11) ist, das einen Heizkörper aufweist, umfassend ein Heizelementgehäuse (22) und ein wärmeleitendes Material (23) sowie zumindest ein elektrisches Widerstandselement (24), insbesondere als Heizwendel, das thermisch an den Heizkörper (11) gekoppelt ist, vorzugsweise zumindest teilweise in den Heizkörper (11) integriert und/oder in den Heizkörper (11) eingebettet ist, und das zur Energieversorgung an eine Energiequelle anschließbar ist und/oder mittels einer Energiequelle mit elektrischer Energie versorgbar ist.

15. Verfahren zum Abgeben flüchtiger Substanzen (6),

mit einer Vorrichtung (1) mit einem Gehäuse (2),

mit einem Behälter (5) für die abzugebende Substanz (6), der zumindest teilweise in das Gehäuse (2) einsetzbar ist,

mit einem Docht (18) als Kapillarelement, der mit der abzugebenden Substanz (6) in Kontakt steht und der zumindest teilweise in dem Behälter (5) angeordnet ist und einen Teil des Behälters (5) bildet und der einen dochtseitigen Substanzabgabebereich (20) aufweist,

mit zumindest einem elektrischen Heizelement (11) im Bereich des dochtseitigen Substanzabgabebereichs (20), mittels dessen ein mit Substanz (6) angereicherter Substanz-Luftstrom, der von der Vorrichtung (1) weg und aus dem Gehäuse (2) heraus strömt, mittels Wärmeübertragung auf den Substanzabgabebereich (20) erzeugbar ist, **dadurch gekennzeichnet,**

**dass** die Dochttemperatur (TD) am dochtseitigen Substanzabgabebereich (20) direkt oder indirekt mit einer Temperaturmesseinrichtung mittels zumindest eines Temperatursensors (13, 14) gemessen wird, und ein elektrisches Dochttemperatur-Messsignal entsprechend der aktuellen Dochttemperatur (TD) während des Betriebs an einem Messwertausgang der Temperaturmesseinrichtung ausgegeben wird,

**dass** die Dochttemperatur (TD) am dochtseitigen Substanzabgabebereich (20) mit einer Steuereinrichtung oder einer Regeleinrichtung gesteuert oder geregelt wird, und

**dass** die Steuer- oder Regeleinrichtung ein Steuer- oder Regelmodul (30) umfasst, an dem ein Dochttemperatur-Sollwert (28) mit einem Sollwertstellglied (27) eingestellt wird und an dem das aktuelle Dochttemperatur-Messsignal als Ist-Dochttemperaturwert (TD) eingegeben wird, und welches die Heizleistung des elektrischen Heizelements (11) mittels einer Vergleichereinheit (26) und eines gespeicherten Steuer- oder Regelalgorithmus variiert und anpasst, um den Dochttemperatur-Sollwert (28) zu erreichen und aufrechtzuerhalten.

**Revendications**

1.  Dispositif pour distribuer des substances volatiles (6),

    avec un boîtier (2),

    avec un récipient (5) pour la substance (6) à distribuer, lequel est au moins partiellement insérable dans le boîtier (2),

    avec une mèche (18) en tant qu'élément capillaire qui est en contact avec la substance (6) à distribuer, et qui est agencée au moins partiellement dans le récipient (5) et forme une partie du récipient (5), et qui présente une région de distribution de substance du côté mèche (20),

    avec au moins un élément chauffant électrique (11) dans la région de la région de distribution de substance du côté mèche (20), au moyen duquel un flux d'air chargé en substance, enrichi en substance (6), qui s'écoule à partir du dispositif (1) et hors du boîtier (2), peut être généré au moyen d'un transfert de chaleur vers la région de distribution de substance (20),

    **caractérisé**

    **en ce que** le dispositif (1) comporte un dispositif de mesure de température pour la mesure directe ou indirecte de la température de la mèche (TD) au niveau de la région de distribution de substance du côté mèche (20), avec au moins un capteur de température (13, 14) et avec une sortie de valeur de mesure, sur laquelle, pendant le fonctionnement, un signal électrique de mesure de température de la mèche correspondant à la température de mèche (TD) mesurée actuellement est fourni,

    **en ce que** le dispositif (1) comporte un dispositif de commande en boucle ouverte ou un dispositif de commande en boucle fermée pour commander la température de la mèche (TD) au niveau de la région de distribution de substance du côté mèche (20), et

    **en ce que** le dispositif de commande comprend un module de commande (30) sur lequel une valeur de consigne de température de la mèche (28) est réglée au moyen d'un organe de réglage de consigne (27) et sur lequel le signal de mesure de température de mèche réel est entré en tant que valeur réelle de température de la mèche (TD), et lequel varie et ajuste de manière algorithmique la puissance de chauffage de l'élément chauffant électrique (11) au moyen d'une unité de comparaison (26) et d'un algorithme de commande mémorisé, afin d'atteindre et de maintenir la valeur de consigne de température de la mèche (28).

2.  Dispositif selon la revendication 1, **caractérisé :**

    **en ce que** le dispositif de mesure de température destiné à simuler et à mesurer indirectement la température de la mèche (TD) au niveau de la région de distribution de substance du côté mèche (20) comporte au moins deux capteurs de température (13, 14), de préférence constitués par des thermistances,

**en ce qu'**au moins un capteur de température, de préférence un ou deux capteurs de température NTC, est agencé en tant que capteur de température de l'élément chauffant (13) pour mesurer la température de l'élément chauffant (TH) sur ou dans l'élément chauffant (11),

**en ce qu'**au moins un capteur de température, de préférence au moins un capteur de température à semi-conducteur, est agencé en tant que capteur de température ambiante (14) pour mesurer l'air ambiant dans le boîtier (2), à distance de l'élément chauffant (11) dans le boîtier (2),

**en ce que** le dispositif de mesure de température comporte au moins un module fonctionnel (25) en tant que module de simulation, de préférence en tant qu'unité de micro-ordinateur, avec une fonction de calcul et/ou une fonction de stockage, auquel les au moins deux capteurs de température (13, 4) sont raccordés pour entrer la température de l'élément chauffant (TH) mesurée et la température de l'air ambiant (TU) mesurée,

**en ce que** la température de la mèche (TD) au niveau de la région de distribution de substance du côté mèche (20) est déterminable indirectement à partir de la température de l'élément chauffant (TH) mesurée et de la température de l'air ambiant (TU) mesurée au moyen du module fonctionnel, de préférence de telle manière que la relation fonctionnelle entre la température de l'élément chauffant (TH) et la température de l'air ambiant (TU) pour la détermination de la température de la mèche (TD)

$$TD = f(TH, TU)$$

a été déterminée expérimentalement à l'avance sur la base d'au moins un dispositif échantillon (1) d'une série de dispositifs et qu'une fonction correspondante, en particulier en tant qu'algorithme et/ou en tant que carte et/ou en tant que courbe caractéristique, est mémorisée dans chaque module fonctionnel (25) de la série de dispositifs, et **en ce que**, pendant le fonctionnement du dispositif (1) et en évaluant la fonction mémorisée, un signal actuel de mesure de température de la mèche, présent en tant que signal de valeur réelle de température de la mèche (TD), est déterminé et est délivré sur ou avec le module fonctionnel (15) formant un simulateur de température de mèche.

3. Dispositif selon la revendication 2, **caractérisé en ce que** la température de la mèche (TD) en fonction de la température de l'élément chauffant (TH) et de la température de l'air ambiant (TU) est déterminée dans le module fonctionnel (25) selon l'équation

$$TD = f(TH, TU) = A * TH + B * TU + C$$

où les variables A, B, C sont déterminées expérimentalement à l'avance avec le au moins un dispositif échantillon (1).

4. Dispositif selon la revendication 2 ou la revendication 3, **caractérisé,**
**en ce que** le au moins un capteur de température de l'élément chauffant (13), de préférence le capteur de température de l'élément chauffant (13) constitué par au moins un capteur de température NTC, est calibrable, notamment pendant la fabrication du dispositif (1), de telle manière :

**en ce que** le au moins un capteur de température de l'élément chauffant (13) à calibrer est combiné, dans un mode de calibration, avec le capteur de température ambiante (14) préalablement calibré, en particulier avec un capteur de température à semi-conducteur en tant que capteur de température ambiante (14) préalablement calibré,

**en ce que** l'élément chauffant (11) est chauffé à une pluralité de valeurs de température de calibration, qui sont mesurées avec le capteur de température ambiante (14) préalablement calibré, et

**en ce que** les valeurs de capteur électriques correspondantes, qui sont déterminées en combinaison, de préférence des valeurs de capteur NTC, sont affectées aux valeurs de température de calibration et sont mémorisées dans une mémoire du module fonctionnel (25), de préférence avec une courbe caractéristique interpolée, très préférablement avec une courbe caractéristique NTC interpolée pour l'ensemble de la plage de mesure.

5. Dispositif selon l'une des revendications précédentes, **caractérisé**
**en ce que** le module de commande comporte un algorithme de commande, de préférence sous la forme d'un régulateur PID (30) avec une combinaison d'une réponse proportionnelle et d'une réponse intégrale et d'une réponse différentielle, la valeur de consigne prédéterminée de température de la mèche (28) étant comparée avec la valeur réelle de température de la mèche (TD) dans une unité de comparaison (26) et une grandeur d'actionnement (31) étant formée côté sortie conformément à l'algorithme de commande et à des paramètres de régulation déterminés expérimentalement à l'avance, et en ce que la grandeur d'actionnement (31) est appliquée à un actionneur commandable (32) pour l'élément chauffant (11), de préférence de telle manière que l'actionneur est au moins un thyristor sous la forme d'un triac avec commande par angle de phase dans l'alimentation en courant alternatif de l'élément chauffant électrique (11), de sorte que la commande par angle de phase du triac et ainsi la puissance de chauffage de l'élément chauffant (11) est variable de manière commandée avec la grandeur d'actionnement (31) afin d'atteindre et de maintenir la valeur de consigne de température de la mèche (28).

6. Dispositif selon la revendication 5, **caractérisé en ce que** l'algorithme de commande est activable de manière discontinue à des intervalles de temps prédéterminés, de préférence dans la plage de secondes, pour l'évaluation d'incréments détectés de la rétroaction de la commande en boucle fermée.

7. Dispositif selon l'une des revendications précédentes, **caractérisé en ce qu'**un dispositif de réglage actionnable manuellement, de préférence sous la forme d'un interrupteur et/ou d'un bouton-poussoir (9), pour la mise en marche et l'arrêt et/ou pour le réglage de différentes valeurs de consigne de température de la mèche (28) est disposé sur le boîtier (2).

8. Dispositif selon l'une des revendications précédentes, **caractérisé en ce que** le dispositif (1) comporte un module radio, de préférence un module WLAN (15) en tant que module radio, pour une connexion de données bidirectionnelle et sans fil entre le dispositif (1) et un dispositif d'affichage et de commande externe, de préférence un smartphone avec une application associée.

9. Dispositif selon la revendication 8, **caractérisé en ce qu'**une valeur de consigne de température de la mèche (28) réglée actuellement et/ou une valeur réelle actuelle de température de la mèche (TD) et/ou une durée d'allumage réglée actuellement du dispositif et/ou, en option, d'autres informations peuvent être affichées sur un écran du dispositif d'affichage et de commande.

10. Dispositif selon la revendication 8 ou la revendication 9, **caractérisé en ce que** des temps de mise en marche sont préréglables avec une fonction minuterie et/ou **en ce qu'**une valeur de consigne de température de la mèche (28) adaptée à une substance (6) devant être évaporée actuellement est réglable avec l'unité d'affichage et de commande du dispositif (1) via la connexion de données sans fil, de préférence de telle manière qu'un code indiqué sur un emballage de substance ou sur un récipient de substance (5), en particulier un code QR, est entré manuellement au moyen d'un clavier ou est lu au moyen d'un scanner, et/ou **en ce que** des temps de mise en marche peuvent être préréglés avec une fonction minuterie,.

11. Dispositif selon l'une des revendications précédentes, **caractérisé**

   **en ce que** l'élément chauffant (11) est annulaire, avec une ouverture centrale de réception de mèche (21) pour la mèche (18), dans laquelle la mèche (18) s'étend au moins partiellement avec sa région de distribution de substance (20), et
   **en ce que** la région de distribution de substance (20) de la mèche (18) est disposée dans le boîtier (2) dans la région d'une ouverture de sortie de flux d'air chargé en substance (10), à partir de laquelle le flux d'air chargé en substance s'écoule vers l'extérieur hors du boîtier (2).

12. Dispositif selon la revendication 11, **caractérisé en ce que** la région de distribution de substance du côté mèche (20) est formée par une extrémité libre de la mèche, qui dépasse du récipient (5), en particulier d'une ouverture de récipient (16), de telle manière que l'élément chauffant (11) est affecté à la mèche (18) dans la région de l'extrémité libre de la mèche.

13. Dispositif selon l'une des revendications précédentes, **caractérisé en ce que** le dispositif (1) est conçu comme composant enfichable avec des contacts enfichables (8) faisant saillie hors du boîtier (2), lesquels contacts enfichables (8) sont enfichables dans une prise électrique pour le maintien et l'alimentation en énergie électrique, de préférence avec une tension alternative.

14. Dispositif selon l'une des revendications précédentes, **caractérisé en ce que** le au moins un élément chauffant est un élément chauffant électrique (11) qui comporte un corps chauffant, comprenant un boîtier d'élément chauffant (22) et un matériau conducteur de chaleur (23) ainsi qu'au moins un élément de résistance électrique (24), en particulier sous forme de spirale chauffante, lequel est couplé thermiquement au corps chauffant (11), de préférence au moins partiellement intégré dans le corps chauffant (11) et/ou incorporé dans le corps chauffant (11), et qui est raccordable, pour l'alimentation en énergie, à une source d'énergie et/ou qui peut être alimenté en énergie électrique au moyen d'une source d'énergie.

15. Procédé de distribution de substances volatiles (6),

avec un dispositif (1) avec un boîtier (2),
avec un récipient (5) pour la substance (6) à distribuer, lequel est au moins partiellement insérable dans le boîtier (2),
avec une mèche (18) en tant qu'élément capillaire qui est en contact avec la substance (6) à distribuer, et qui est agencée au moins partiellement dans le récipient (5) et forme une partie du récipient (5) et qui présente une région de distribution de substance du côté mèche (20),
avec au moins un élément chauffant électrique (11) dans la région de la région de distribution de substance du côté mèche (20), au moyen duquel un flux d'air chargé en substance, enrichi en substance (6), qui s'écoule à partir du dispositif (1) et hors du boîtier (2), peut être généré au moyen d'un transfert de chaleur vers la région de distribution de substance (20),
**caractérisé**
**en ce que** la température de la mèche (TD) au niveau de la région de distribution de substance du côté mèche (20) est mesurée directement ou indirectement au moyen d'un dispositif de mesure de température au moyen d'au moins un capteur de température (13, 14), et un signal électrique de mesure de température de la mèche correspondant à la température de mèche (TD) actuelle est délivré sur une sortie de valeur de mesure du dispositif de mesure de température pendant le fonctionnement,
**en ce que** la température de la mèche (TD) au niveau de la région de distribution de substance du côté mèche (20) est commandée au moyen d'un dispositif de commande en boucle ouverte ou d'un dispositif de commande en boucle fermée, et
**en ce que** le dispositif de commande comprend un module de commande (30) sur lequel une valeur de consigne de température de la mèche (28) est réglée au moyen d'un organe de réglage de consigne (27) et sur lequel le signal de mesure de température de mèche actuel est entré en tant que valeur réelle de température de la mèche (TD), et lequel varie et ajuste la puissance de chauffage de l'élément chauffant électrique (11) au moyen d'une unité de comparaison (26) et d'un algorithme de commande mémorisé, afin d'atteindre et de maintenir la valeur de consigne de température de la mèche (28).

Fig. 1

Fig. 2

16

Fig. 3

Fig. 4

Fig. 5

EP 4 301 422 B1

**EP 4 301 422 B1**

**Patent documents cited in the description**

- WO 2017215726 A1
- WO 9858692 A1

- WO 2007138246 A2